# EUROPEAN PATENT SPECIFICATION

(11) **EP 1 883 428 B1**
(45) Date of publication and mention of the grant of the patent: **15.07.2009**
(21) Application number: 06757854.2
(22) Date of filing: 19.05.2006
(51) Int. Cl.: A61K 49/04

(54) **CONTRAST AGENTS**
KONTRASTMITTEL
AGENTS DE CONTRASTE

(30) Priority: 20.05.2005 NO 20052428
(43) Date of publication of application: 06.02.2008
(73) Proprietor: GE HEALTHCARE AS, Nydalen 0401 Oslo (NO)
(72) Inventor: AXELSSON, Oskar, 221 84 Lund (SE); BJERKNES, Kathrin, 0401 Oslo (NO)
(74) Representative: Flechsler, Insa
(86) International application number: PCT/NO2006/000187
(87) International publication number: WO 2006/123937

(56) References cited:
- WO-A-95/26205
- WO-A-96/11023
- WO-A2-03/075961
- WO-A2-20/05051435
- US-A- 6 001 333

## Description

The present invention relates to liposomes susceptible for uptake by Kupffer cells and to pharmaceuticals containing such liposomes. The liposomes comprise particles of coated cores of the metallic element of tungsten or of tungsten in mixture with other metallic elements as the contrast enhancing material. The invention also relates to the use of such pharmaceuticals as contrast agents in diagnostic imaging, in particular in X-ray imaging of liver tumours, and to contrast media containing such liposomes.

All diagnostic imaging is based on the achievement of different signal levels from different structures within the body. Thus in X-ray imaging for example, for a given body structure to be visible in the image, the X-ray attenuation by that structure must differ from that of the surrounding tissues. The difference in signal between the body structure and its surroundings is frequently termed contrast and much effort has been devoted to means of enhancing contrast in diagnostic imaging since the greater the contrast between a body structure and its surroundings the higher the quality of the images and the greater their value to the physician performing the diagnosis. Moreover, the greater the contrast the smaller the body structures that may be visualized in the imaging procedures, i.e. increased contrast can lead to increased spatial resolution and thereby achieving a safer detection of the target for the diagnostic procedure.

The diagnostic quality of images is, for a given spatial resolution, strongly dependent on the inherent noise level in the imaging procedure, and the ratio of the contrast level to the noise level can thus be seen to represent an effective diagnostic quality factor for diagnostic images. The ratio of the signal level and the noise level is usually denoted signal to noise ratio, abbreviated SNR.

Achieving improvement of the diagnostic quality factor has long been and still remains an important goal. In techniques such as X-ray, magnetic resonance imaging (MRI) and ultrasound, one approach to improve the diagnostic quality factor has been to introduce contrast enhancing materials, contrast agents, into the body region to be imaged.

Thus in X-ray for example early examples of contrast agents were insoluble inorganic barium salts which enhanced X-ray attenuation in the body zones into which they distributed. More recently the field of X-ray contrast agents has been dominated by soluble iodine containing compounds and specifically iodinated aryl compounds such as those marketed by Amersham Health AS under the trade names Omnipaque™ and Visipaque™.

Work on X-ray contrast agents having heavy metals as the contrast enhancing element has to a great extent concentrated on aminopolycarboxylic acid (APCA) chelates of heavy metal ions. Recognising that effective imaging of many body sites requires localization at the body sites in question of relatively high concentrations of the metal ions, there have been suggestions that polychelants, that is substances possessing more than one separate chelant moiety, might be used to achieve this. Further work has been concentrated on the use of multinuclear complexes that are complexes wherein the complexed moiety itself comprises two or more contrast enhancing atoms, see Yu, S.B. and Watson, A.D. in Chem. Rev. 1999, 2353-2377. Thus, for X-ray or ultrasound the complexes would comprise two or more heavy metal atoms and for MRI the complex would contain two or more metal atoms with paramagnetic properties. Yu and Watson also discuss use of metal-based X-ray contrast media. Tungsten powder is noted for use as an X-ray contrast additive in embolic agents used in the treatment and preoperative embolisation of hypervascular tumors. However, they find it likely that general intravascular use of heavy metal complexes is limited by safety concerns and dosage requirements.

X-ray contrast agents for parenteral administration are mainly hydrophilic of nature and have approximately the same extracellular biodistribution and are preferably renally excreted. Various attempts are made to achieve organ specific X-ray contrast agents that accumulate in organs and cells of the body and X-ray contrast agents which have extended residence time in the blood pool (blood pool contrast agents) and which can be administered parenterally. Iodinated aryl based X-ray contrast agent for example has been linked to macromolecular substrates such as starch in order to improve their vascular half-life. Potential liver contrast agents based on biodegradable particles are proposed in e.g. Kao et al, Academic Radiology; 2003; 10; 475 - 483, WO-A-8900988 and WO 9007491. Liposomes containing ionic or non-ionic iodinated aryl compounds have also been suggested, see e.g. WO-A-8809165 and US 5,676,928. In the later years targeting moieties such as specific vectors binding to receptors at the target organs or cells have been proposed.

PCT/NO2004/00036 proposes particles with a core of the metallic element tungsten optionally together with other metallic elements and being coated with a coating layer. The particles described in this document should preferable be below the kidney threshold of about 6 to 7 nm to ensure excretion through the kidneys. The coating could be monomeric and polymeric and provide particles with a short half-life in vivo. Surface coatings with targeting moieties embedded such as antibodies are also proposed for the targeting of various body organs and structures including tumours and macrophages.

In oncology, early diagnosis of liver tumours such as hepatomas and metastatic spread to the liver are major causes of death in the world. There is a continuing need for methods and products to help in the early diagnosis of cancer. Cancer tissues in general have different vascularity from healthy tissues and may be detected as an area of modified contrast. However, X-ray examination of the liver will typically require high amounts of iodinated contrast agent and injection of contrast agent containing ca. 9 g iodine may be required, see WO -A- 8809165. The Kupffer cells reside in the liver and will take up and initially break down particles. Kupffer cells are not present or only present to a low extent in liver tumour tissue. Hence there is a possibility to identify cancerous liver tissue as tissue that give no or very low signal in X-ray examination of the liver after administration of a suitable X-ray contrast agent.

None of the attempts to provide specific X-ray contrast agents for imaging of liver tumours has resulted in commercial products. Problems encountered in this regard has been insufficient contrast in the target organ or structure and the need for very high doses of contrast agents e.g. in the form of conventional iodinated X-ray contrast agents enclosed in liposomes which may lead to adverse reactions. It has hence been difficult to achieve a satisfactory signal to noise ratio (SNR) sufficient to secure a safe and accurate diagnosis in particular of small lesions.

It has now surprisingly been found that liposomes being susceptible for uptake by Kupffer cells can be provided that provide sufficient contrast in X-ray contrast examination for the identification of tumour tissue. Such compounds are liposomes carrying particles comprising a core of the metallic element tungsten optionally together with other metallic elements and being coated with a coating layer. The tungsten containing particles are predominantly contained in the interior of the liposomes. Liposomes comprising such tungsten containing particles surprisingly provide sufficient uptake of contrast agents by the Kupffer cells to achieve sufficient contrast and SNR.

The invention will now be described in further details. The various embodiments are also specified in the attached claims and form part of the entire description of the invention.

Coated particles comprising tungsten containing cores are enclosed in PCT/NO2004/00036.

It should be noted that the terms core, metallic core and tungsten (containing) core are used interchangeably in the further document and that the particles comprising the core and a coating are interchangeably denoted particle, metallic particle and tungsten (containing) particle.

The present invention is based upon the finding that the liposomal contrast agents can be made such that the liposomes enclose a sufficient amount of particles and be of suitable size to be taken up by the Kupffer cells.

Liposomes of a size greater than 85 nm will be removed from the blood compartment by opsonisation and receptor mediated phagocytosis by the Kupffer cells and will be transferred to the liver where they are broken down and finally excreted.

The liposomes to be used as contrast agents must therefore be of a size sufficiently to be effectively recognized and taken up by the Kupffer cells. Hence, the diameter of the liposomes is from 200 nm to 1000 nm (1 µm).

The liposomes provided will have a sufficient encapsulation capacity, such as 5 gW/g lipid to 50 gW/g lipid and preferably about 20 gW/g lipid in order to function as an efficient transport means for the tungsten containing particles. W denotes tungsten and lipid refers to the liposome membrane forming lipids. High tungsten to lipid ratio is also beneficial from a safety point of view. Generally therefore, liposomes with a relatively large average diameter of at least 100 nm is preferred since the interliposomal compartment and thereby the encapsulation capacity increases with a power of 3 with the increase of the diameter of the liposome. The upper diameter should generally not exceed 1000 nm (1µm) since larger liposomes may get stuck in and block blood flow in the capillaries, and these are also less stable.

The liposomes can be unilamellar or multilamellar of constitution, however it is preferred that the liposomes contain few bilayers of amphiphilic material, preferably the liposomes are unilamellar or bilamellar, however liposomes containing three to five bilayers of amphiphilic material can also be used.

Hepatocytes, which constitute about 80% of the liver mass, also take up liposomes to some extent. To promote uptake of the Kupffer cells and to discriminate against uptake by the hepatocytes, the incorporation of substances into the liposomes that trigger phagocytosis is preferred. Examples of such substances are lipopolysaccharides (LPS) and lipoteichoic acids (LTA). Such substances are preferably incorporated into the membrane of the liposomes.

Further examples of membrane forming lipids include lipopeptides, lipophilically derivatised carbohydrates e.g. carrying one or more fatty acyl groups, sphingolipids, glycolipids, glycerolipids, cholesterol and phospholipids.

Examples of phospholipids are phosphatidic acids, phosphatidyl cholides, phosphatidylserines, phosphatidylglyceroles, phosphatidylethanolamides, phosphatidylinositoles, cardiolipids and corresponding lyso analoges thereof.

Phospholipids having relatively long-chained lipophilic fatty acid residues are preferred. The fatty acid residues should have lipophilic residues of at least 10 member chains, e.g 12 carbon atom chains which can be straight or branched chains, e.g. alkyl or alkenyl residues. Preferably the lipophilic residue should contain at least 16 chain members and should more preferably contain 18 chain members, e.g. palmitic acid or stearic acid residues. When the number of carbon atoms in a fatty acid residue is below 14, the ability of the liposomes to hold the internal particle containing phase which is usually an aqueous phase, is relatively low, and the stability of the liposomes in blood after administration is low. On the other hand, where the number of carbon atoms in the fatty acids residues is 28 or more, the biocompatibility becomes low and a high temperature is necessary during the production of the liposomes.

It is further a preferred option that the liposomes should have a negatively charged surface to further promote uptake by the Kupffer cells and to counteract clustering of the liposomes. Liposomes of the same surface charge will repel each other. The liposomes should therefore preferably be prepared from a mixture of neutral lipids such as phospholipids and negatively charged lipids such as phospholipids.

The charged groups must be in their ionic form at the pH of the environment where the compound is used. Most importantly they must be in charged form at physiological pH, in particular at the pH of blood.

The phospholipids can be synthetic phospholipids or phospholipids derived form natural sources. Preferred neutral phospholipids include for example neutral glycophospholipids for example partially or fully hydrogenated naturally occurring phospholipids e.g derived from soy bean or egg yolk or synthetic phospholipids, particularly semi-synthetic or synthetic dipalmitoyl phosphatidylcholine (DPPC), dimyristoyl phosphatisylcholine (DMPC), dilauroyl phosphatidylcholine (DLPC), DOPC - dioleyl phosphatidylcholine (DOPC), phosphatidylcholine (PC), distearoyl phosphatidylcholine (DSPC) and sphingomyelin (SM).

Preferred negatively charged phospholipids include for example phosphatidylserine, for example a partially or fully hydrogenated naturally occuring phosphatidylserine from soy bean or egg yolk or semi-synthetic or synthetic dipalmitoyl phosphatidylserine (DPPS) or distearoyl phosphatidylserine (DSPS), phosphatidyl glycerol for example partially or fully hydrogenated naturally occuring phosphatidylglycerol from soy bean or egg yolk or semi-synthetic phosphatidylglycerol, particularly semi-synthetic of synthetic dipalmitoyl phosphatidylglycerol ((DPPG) or disteaoryl phosphatidylglycerol (DSPG); phosphatidyl inositol, for example a partially or fully hydrogenated naturally occuring phosphatidylinositol from soy bean or egg yolk or semi-synthetic phosphatidyl inositol, particularly semi-synthetic or synthetic dipalmitoyl phosphatidylinositol (DPPI) or distearoyl phosphatidylinositol; phosphatidic acid, for example a partially or fully hydrogenated naturally occuring phosphatidic acid from soy bean or egg yolk or semi-synthetic phosphatidic acid, particularly semi- synthetic or synthetic dipalmitoyl phosphatidic acid (DPPA) or distearoyl phosphatidic acid (DSPA). Although such charged phospholipids are commonly used alone, more than one charged phospholipid may be used.

The tungsten containing particles for incorporation into the liposomes are particles comprising a core and a coating layer. The particle size can vary in a range but should be at least 3 nm, e.g. from 3 nm to 100 nm. Particles of a size to below 20 nm are further preferred for this purpose, e.g. particles from 3 to 20 nm.

The particles consist of a core and a coating substantially completely covering the core. The core of the particle contains tungsten in its metallic form or tungsten in mixture with other suitable metallic elements. Preferably the tungsten content is between 20 and 100 weight%, more preferably between 50 and 100%, and even more preferably of 85 to 100 weight% and particularly preferably between 95 and 100 weight%. Cores of about 100% tungsten are generally preferred.

Metallic tungsten has a relatively high X-ray attenuation value, a low toxicity and is available at an acceptable price.

Introducing other metallic elements in addition to the tungsten in the core can provide improved properties to the core e.g. can improve the stability, monodispersity and can facilitate the synthesis and/or the rate of formation of the metal core. Preferably 5 to 15 weight% of rhenium, iridium, niobium, tantalum or molybdenum either as a single element or as mixtures of elements are feasible additives, most preferred are rhenium and iridium. All these elements are miscible with tungsten and small amounts of rhenium and/or iridium improve the low temperature plasticity of the metallic core.

It is important that the metallic cores which provide the attenuating properties to the particles are of a sufficient size with regard to this property taking into consideration the preferred total size of the particle. The particle must hence contain an as high amount as possible of metal atoms to provide the desired attenuating properties.

Since the tungsten containing core is reactive to a greater or lesser extent, the metallic core must be coated in order to passivate the reactive surface. The properties of the coating should provide a protection to the metallic core such that the core does not react e.g. ignite when exposed to air or react when formulated for in vivo use or react in the in vivo environment. The coating must be relatively thin in order to optimize the amount of contrast enhancing material for a given particle size. The thickness of the coating should preferably be between 1 and 5 nm. The binding between the metal core and the coating should also be sufficiently strong to avoid disintegration between the metallic core and the coating.

In the liver the liposomes are taken up by the Kupffer cell and phagocytosed. The particles are degraded relatively slowly and excreted from the body. Some leakage of the core metals such as tungsten is acceptable since tungsten is of relatively low toxicity and released metal will rapidly be excreted through the kidneys.

The coating layer can be monomeric or polymeric and should preferably be a polymeric coating layer. In those cases wherein monomeric coating provides particles of sufficient stability, those are also favourable.

Tungsten containing cores having a monomeric coating are described in PCT/NO2004/00036.

The monomeric coating should preferably comprise a layer of non-metallic material with a hydrophilic part facing the aqueous surroundings, an inert middle layer and a metal binding part facing the metal core, comprising at least a fraction of molecules that are hydrophilic and preferentially each molecule should have at least one hydrophilic group. The coating should at the same time cover the core surface (e.g. the tungsten core surface) densely enough to passivate it. The passivation takes place on the surface of the core where there is an electron transfer between the metal coordinating group and the surface of the core. Examples of metal coordinating groups are groups A in the formula An-Lo-Mp presented below. In a preferred aspect the coating is a mono-layer coating, meaning that the thickness of the coating is only one single molecule. Monomeric coating has the benefit that the coating layer can be made thin and with well defined properties. Albeit polymeric coatings can be said to consist of single molecules as well, they are distinct from monomeric coatings in their mode of binding to the core. The monomeric coatings bind in a radial orientation with the metal binding part facing the core, the middle part extending radially and the hydrophilic part facing the aqueous environment. A polymeric coating winds across the metal surface and interacts with the metal core at multiple points along the chain. The efficacy of the particles depends on that the tungsten core of the particles constitutes the highest possible fraction of the particle.

The mono-layer coating is preferably built according to the general formula Aₙ-Lₒ-Mₚ, where A is one or more metal coordinating groups preferably selected from Table 1, L is absent or is one or more linking groups preferably selected from Table 2, and M is one or more charged and hydrophilic groups preferably selected from Table 3. The linking group preferably comprises any number of fragments from Table 2 arranged linearly, branched or in one or more rings. The branching may be towards the A group side to create multidentate coatings or it may branch towards the M group to create a higher degree of hydrophilicity. Branching in both directions is also an option. Linking fragments from Table 2 may be combined to phenyl rings or aromatic or non-aromatic heterocyclic groups. n is any positive integer and preferably from 1 to 10 or more preferably from 1 to 4. o is zero or any positive integer and preferably from 1 to 10 or more preferably from 1 to 2. p is any positive integer and preferably from 1 to 10 or more preferably from 1 to 4. The dotted line for the groups A in Table 1 indicates a bond to the tungsten element, a bond to an H-atom, a bond to the L-group, a bond to another A-group or a bond to the M group when o is zero. The dotted line for the groups L indicates a bond to the A group, a bond to an H-atom, a bond to another L-group or a bond to the M-group. The dotted line for the groups M indicates a bond to the L group, a bond to an H-atom, a bond to another M-group or a bond to the A-group when o is zero.

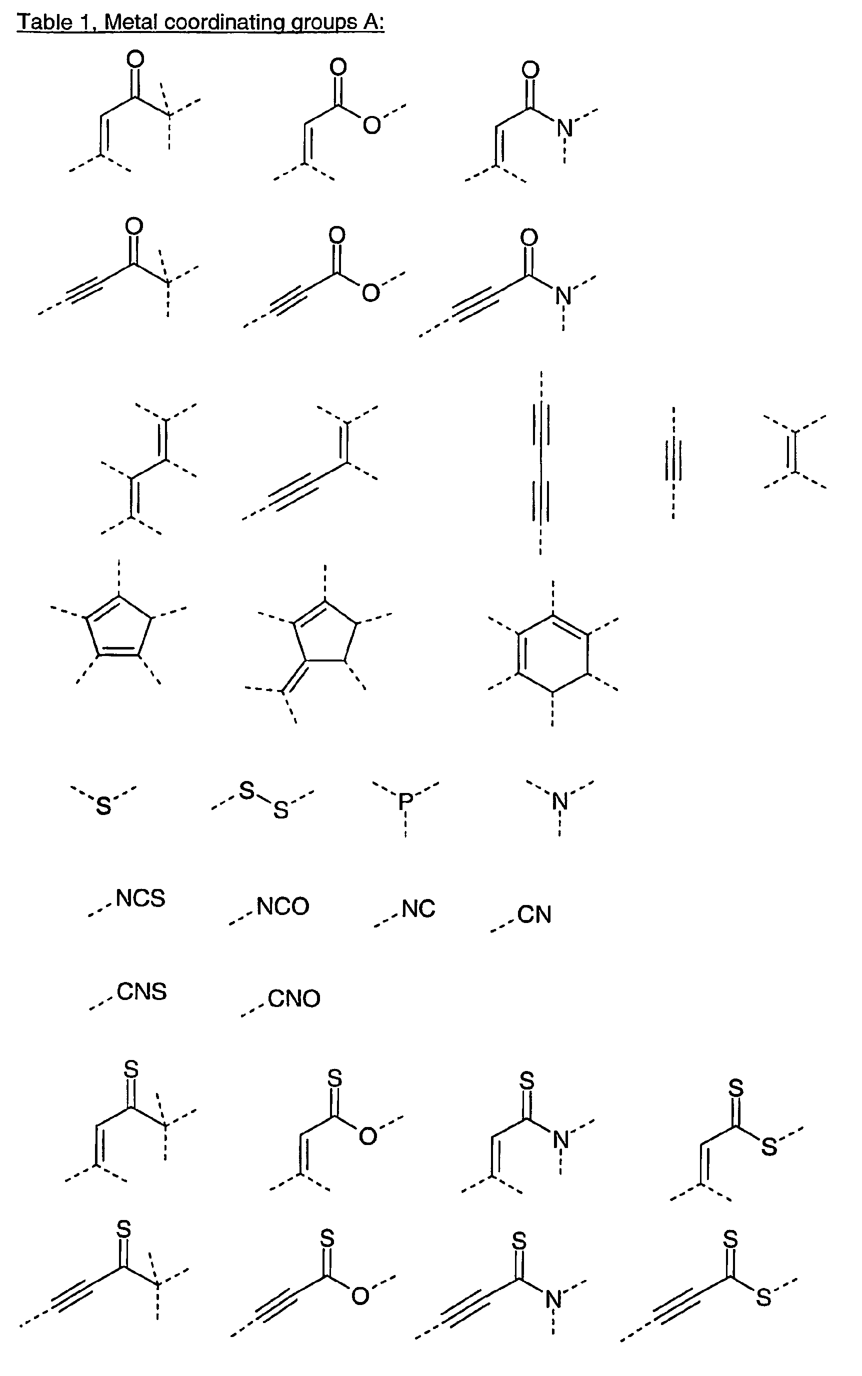

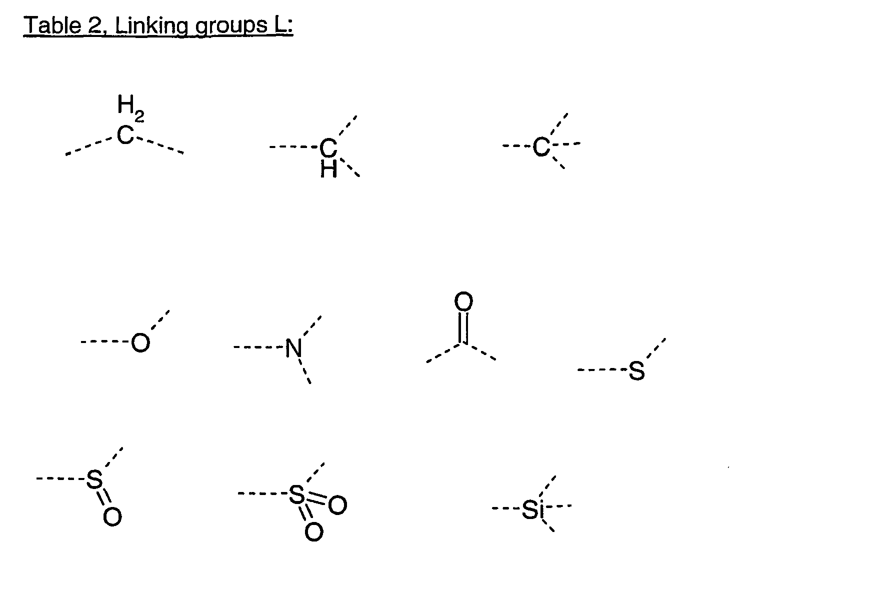

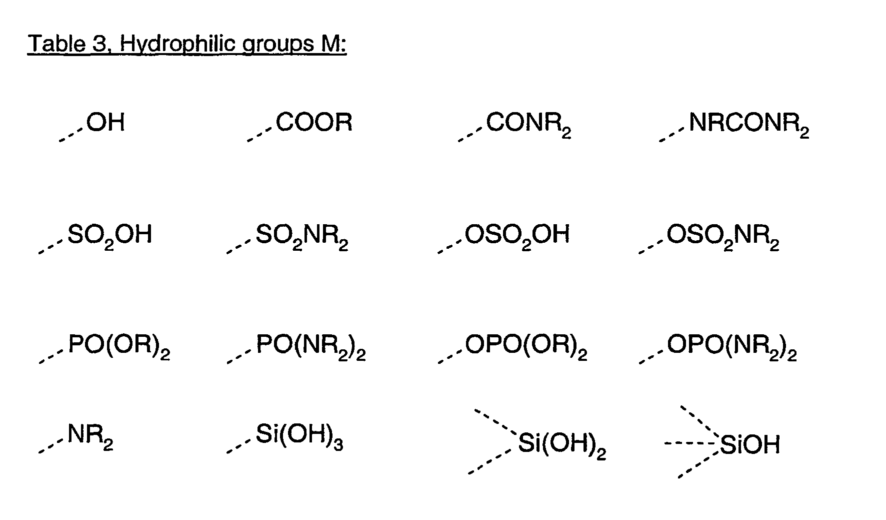

The R - groups are independently any group(s) selected from the group of H and a C₁-C₆ alkyl group optionally substituted by one or more -OH groups and where one or more of the C-atoms of the C₁-C₆ alkyl group may be replaced by an ether group.

The polymeric coating layer comprises a layer of any polymeric material suitable for pharmaceutical use containing a small number of charged groups per nanoparticle and being hydrophilic. The coating should cover the tungsten surface densely enough to passivate it. The polymeric surface layer can be covalently bound to the metallic core surface or adsorbed and held by non-covalent forces. As described above for the monomeric coating, it is preferred that the coating layer is as thin as possible and at the same time providing the necessary passivation of the tungsten core surface. The polymer can be a natural or synthetic homopolymer or copolymer. Numerous polymers are available for the purpose and the skilled artisan will be able to choose suitable polymers known from the state of art. Useful classes of polymers comprise polyethers (e.g PEG and optionally branched), polyacetals, polyvinylalchohols and polar derivatives thereof, polyesters, polycarbonates, polyamides including aliphatic and aromatic polyamides and polypeptides, classes of carbohydrates such as starch and cellulose, polycyanoacrylates and polycyanometacrylates, provided that the polymers include a minimum of charged groups and most preferable also are hydrophilic. Polymers made of acrylic acid monomers are specifically preferred. In order to obtain a layer with a controlled and suitable number of charged groups, copolymers are also preferred wherein the copolymer can contain 2 or more momomeric entities or blocks. At least one of the monomers may provide charged groups to the polymer coating. The charge increases the water-solubility and reduces the risk of particle aggregation.

Examples of suitable monomers to be used to form the polymer coating are:

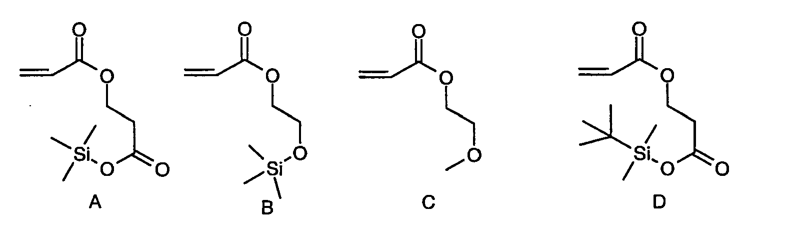

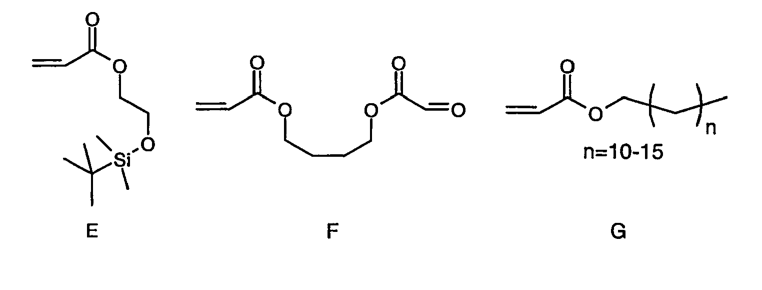

Use of monomer F forms a cross-linked polymer.

The monomers of the coating material may further comprise lipophilic groups in the form of at least a fraction of lipophilic groups in the polymeric coating layer.

Contrast media are frequently administered parentally, e.g. intravenously, intra-arterially or subcutaneously. Contrast media can also be administered orally or via an external duct, e.g. to the gastrointestinal tract, the bladder or the uterus. Suitable carriers are well known in the art and will vary depending on e.g. the administration route. The choice of carriers such as excipients and solvents is within the ability of the skilled artisan. Usually aqueous carriers are used for dissolving or suspending the pharmaceutical, e.g. the contrast agent to produce contrast media. Various aqueous carriers may be used such as water, buffered water, saline, glycine, hyaluronic acid and the like.

In one embodiment the invention comprises pharmaceuticals comprising the liposomes as hereinbefore described together with a pharmaceutically acceptable solvent or excipient, and specifically such agents for use as diagnostic agents, in particular for use as X-ray contrast agents.

The viscosity and osmolality of the particles in solution must also be taken into account. The viscosity should generally be lower than 40 mPas to provide ease of administration. To avoid adverse effects, in particular adverse effects connected to the osmolality, the solution of the particles for administration should be slightly hyperosmolal or about iso-osmolal with blood. A suitable osmotic pressure of the injectable pharmaceutical can be achieved by addition of a balanced amount of suitable excipients, e.g. of sorbitol. The pH of the preparation should be in the physiological acceptable area. The pH can be adjusted with an acceptable buffer, e.g. with TRIS buffer.

It will be possible to formulate solutions containing the liposomes of the invention having from about 1.0 to about 4.0 g tungsten/ml solution, more specifically from 1.5 to about 3.0 g tungsten/ml solution and most specifically about 2.2 g tungsten/ml solution.

In further embodiments the invention comprises a method of diagnosis and a method of imaging where the liposomes are administered to a human or animal body. The body is examined with a diagnostic device and data are compiled from the examination. The data can be further processed if needed to facilitate that the data can be used to create an image and to reach to a diagnosis. The data can be used for the visualisation and identification of liver tumours.

Liver tumours are identified as areas in the liver that are not enhanced by the contrast medium. This is because the contrast agents are taken up by the liver Kupffer cells. Kupffer cells are not present or only present to a very low extent in liver tumour tissue. Active uptake of the particles of the diagnostic agents of the invention by the Kupffer cells would then make it possible to identify liver tumours as areas of the liver that is not contrast enhanced. For liver tumour diagnosis, it is preferred to administer the diagnostic agent of the invention as a single bolus dose.

Although if may be possible to use plain X-ray imaging in the signal uptake in the methods of the invention at least for large lesions, X-ray Computed Tomography (CT) is highly preferred. The molar extinction of tungsten for CT relevant X-rays (120 kV) is 5500 Hounsfield units/mol (HU). A modern CT has a noise level of about 15 HU for a reasonable resolution, and it is possible to identify lesions in the millimetre range.

Liposomes containing tungsten particles can be prepared by following the directions in a) and b) below.

### a) Preparation of tungsten particles

Generally, the coated particles are prepared by thermally decomposing a source of tungsten (0), e.g. tungsten hexacarbonyl, W(CO)₆, in the presence of the chemical compounds constitution the coating layer.

Particles containing tungsten cores coated by a hydrophilic layer can be produced by mixing tungsten hexacarbonyl, W(CO)₆ with the molecules that will constitute the coating and wherein all reactive sites are protected and a reduction agent under inert gas is used. After the reduction is completed the protection groups are removed. Alternatively, the reduction can also be performed in aqueous solution or in reverse micelles.

The polymer coated particles are prepared by thermally decomposing a source of tungsten (0), e.g. tungsten hexacarbonyl, W(CO)₆, in a high-boiling, dried and deoxygenated solvent in the presence of one or more of the monomers. A thermally induced polymerization of the monomers takes place, covering the tungsten particles formed from the decomposition, with a polymeric coating. When the monomers comprise silylether-protected polar groups (-OH, -COOH) the protecting groups are cleaved in aqueous solution to yield the hydrophilic polymer coated particles.

Dry solvents should generally be used. Hygroscopic solvents (diglyme, triglyme) should be percolated through alumina and stored over molecular sieves. All solvents should be deoxygenated by letting a stream of argon bubble through the solvent for 25-30 minutes before they are used in the reactions. The choice of solvent for this process is critical since there are several criteria to be fulfilled. One is the ability to dissolve the starting materials and at the same time keep the final polymer coated particles in solution. The polyethers di- and triglyme are particularly useful here. The high boiling point of triglyme in particular, will allow the temperature to reach the level where the last carbon monoxide molecules leave the particles. Other useful solvents would be diphenyl ether and other inert high-boiling aromatic compounds. Also trioctyl phosphine oxide (and other alkyl analogs), trioctyl phosphine (and other alkyl analogs), high boiling amides and esters would be useful.

Another important process parameter is the ability to control the tendency of W(CO)₆ to sublimate out of the reaction mixture. This can be achieved by mixing in a small fraction of a lower boiling solvent to continuously wash back any solid tungsten hexacarbonyl from the condenser or vessel walls. Cyclooctane and n-heptane would be good choices when used in 5 to 15 % volume fraction.

For the work-up of the particles, precipitation by the addition of pentane or other low-boiling alkanes would be convenient. A low boiling point solvent is advantageous when the particles are to be dried.

The preparation and work-up procedures are further described in example 1 and 2 below and in PCT/NO2004/00036.

### b) Preparation of liposomes

Liposomes enclosing the metal containing particles can be prepared by methods known from the state of art. There are several processes for preparation of liposomes, i.e. extruding through several filters, thin film hydration method, reverse phase evaporation method etc. These methods are illustrated in Examples 7 to 9 below.

### Examples

The invention will hereinafter be further illustrated with the non-limiting examples. All temperatures are in °C. The tungsten content in the particles was determined by X-ray fluorescence spectroscopy. Dynamic Light Scattering was used to determine particle size of one of the preparations.

### Example 1

### Preparation of a polymer coated tungsten nanoparticle comprising monomers B and C

In a round-bottomed flask fitted with a magnetic stirrer and a condenser was put: Tungsten hexacarbonyl W(CO)₆ (500mg, 1.4 mmol), ethyleneglycol methylether acrylate (C) (390 mg, 3.0 mmol), and trimethylsilyl protected 2-carboxyethyl acrylate (B) (120 mg, 0.55 mmol). The condenser was fitted with a septum and several vacuum / argon cycles were applied to deairate the flask and condenser. Deairiated diglyme (30 ml) and heptane (2 ml) were added through the septum with a syringe. The reaction mixture was heated to reflux under an argon atmosphere. After 3 h, the reaction mixture, now a black solution with small amounts of black precipitate, was cooled to room temperature, poured on deairated pentane (60 ml) and centrifuged. The precipitate was washed with pentane and dried in vacuum.
Yield: 430mg dark grey powder. X-ray fluorescence spectroscopy analysis showed the tungsten content to be around 60%.
Comments: The heptane is needed to prevent tungsten hexacarbonyl sublimation deposits in the condenser. The trimethyl silyl protection group is spontaneously cleaved in aqueous solutions, yielding the preferred carboxylate G.

The particles have a core of crystalline tungsten covered by a thin coating of co-polymerized C and B. The particles are between 4 and 5 nm.

### Example 2

### Preparation and analysis of polymer coated tungsten nanoparticles comprising monomers B and D

Tungsten hexacarbonyl (440 mg, 1.2 mmol), monomer B (970 mg, 5.0 mmol) and monomer D (300mg, 1.1mmol) were put in a glass flask equipped with a condenser and a magnetic stirrer. The flask and condenser were subjected to several vacuum/ argon cycles leaving an argon atmosphere. Cyclooctane (30 ml) was added with a syringe through a septum at the top of the condenser. The reaction solution was stirred and heated to reflux for 18 h. During the first hours, the solution slowly darkened, eventually becoming black. After completed reaction time, the solution was cooled to room temperature and poured on pentane (50ml). The resulting slurry was centrifuged and the precipitate was washed with pentane and dried in vacuum.
Yield: 400 mg dark powder
Analysis
¹H NMR: broadened resonances appeared at (ppm) 4.3, 4.1, 3.8, 3.5, 2.8, 2.7-2.2,1.8-1.2, 0.8, 0.1.
IR: 1939w, 1852w, 1731vs, 1560m.
XFS: 57 % W
Solubility in water: > 500 mg / ml.

### Example 3

### Preparation and analysis of Polymer coated tungsten, nanoparticles comprising monomers A and C

Tungsten hexacarbonyl (500 mg, 1.4 mmol), monomer A (120 mg, 0.55 mmol) and monomer C (390 mg, 3.0 mmol) were added to the glass flask following the procedure of example 2. Diglyme (30 ml) and heptane (2ml) were added through the condenser. The reaction solution was stirred and then heated to reflux for 3h. Yield: 410 mg dark powder.

### Analysis:

¹H NMR: broadened resonances appeared at (ppm) 4.1, 3.5, 3.2, 2.5-2.2,1.9-1.3. IR: 1995w, 1894w, 1727vs, 1540s.
XFS: 55 % W.
TEM: a micrograph showing particle cores in the size of 3-4 nm was obtained. Degradation experiment: an exponential decrease in absorption over the whole spectrum (300-800 nm). At most, the absorption decreased 22 % in 4.3 h (at 350 nm). Electrophoresis experiment: movement of the particles implied negative charge.

### Example 4

### Preparation and analysis of polymer coated tungsten, nanoparticles comprising monomers A and C

Tungsten hexacarbonyl (1.5 g, 4,3 mmol), monomer C (1.15 g, 9.0 mmol) and monomer A (0.36 g, 1.6 mmol) were added to a glass flask equipped with a condenser and a magnetic stirrer. The flask and condenser were subjected to several vacuum/argon cycles leaving an argon atmosphere. Deairated triglyme (85 ml) and cyclooctane (15ml) were added with a syringe through a septum at the top of the condenser. The mixture was stirred at 150°C for 4 hours. The temperature was then raised to approximately 200°C (reflux) for 30 minutes. The black solution formed was cooled to room temperature, diluted with pentane (1:1) and centrifuged. The precipitation was collected, washed twice with pentane and dried under vacuum. The dried product was stirred in deairated water and titrated with NaOH until dissolution. The resulting solution was freeze-dried. Yield: 950 mg dark grey powder.

### Example 5

### Preparation and analysis of polymer coated tungsten nanoparticles comprising monomer E

Tungsten hexacarbonyl (2.3 g, 6.5 mmol)m and monomer E (7.6 g, 32 mmol) were added to the glass flask following the procedure of example 7. Cyclooctane (100 ml) were added through the condenser. The reaction solution was stirred and then heated to reflux for 60h.

### Analysis:

Size of particles was determined by Dynamic Light Scattering. 99% of the total particle volume belonged to particles having a size between 5.8-7.8 nm.

### Example 6A

### Preparation and analysis of polymer coated tungsten nanoparticles comprising monomer A, C and F

Tungsten hexacarbonyl (1.0 g, 2.8 mmol), triglyme (45 ml) and heptane (3 ml) were put in a glass flask equipped with a condenser and a magnetic stirrer. The flask and condenser were subjected to several vacuum/ argon cycles leaving an argon atmosphere. The slurry was heated and stirred until dissolution. The solution was then heated to 160°C after which a mixture of monomer C (1.8 g, 14 mmol), monomer A (280 mg, 1.3 mmol) and monomer F (280 mg, 1.4 mmol) was added with a syringe through a septum. The solution was stirred at 165-170°C for 3h. After completed reaction time, the solution was cooled to room temperature and poured on pentane (50 ml). The resulting slurry was centrifuged and the precipitate was washed with pentane and dried in vacuum. Yield: 800 mg dark powder. Analysis
¹H NMR: broadened resonances appeared at (ppm) 4.2, 3.5, 3.3, 2.3, 2.0-1.4. IR: 1921w, 1825w, 1727vs, 1534m.
XFS: 47 % W.

### Example 6B

### Tungsten particles with monomeric coating

A solution of a suitable tungsten(0) complex e.g. W(CO)₆ and an alkyl thiol with a masked polar group in the teminal position are mixed in a molar ratio of 1:5 in a high boiling, inert solvent. The mixture is heated to 180-220 degrees Celcius for 10-20 hours. Heptane is added and the dark mixture is centrifuged to collect a dark material. This is resuspended in dichlorometane by sonication and centrifuged again. This is repeated until NMR analysis shows the powder to be free from the high boiling solvent. Usually two times is enough. The polar groups are unmasked either by removal of protecting groups or by chemical modification of the masking groups, e.g. oxidation. The particles can be purified from the unmasking reagent mixture by centrifugation and modification of the solvent strength similar to the first step.

### Example 7

### Preparation of liposomes containing tungsten particles by extruding through filters.

Materials for use in the preparation:
Tungsten aqueous suspension (300 mg/ml) from either of Examples 1 to 6
Lipid mixture e.g. H-EPC/H-EPSNa (11:1) 15 g
H-EPC - Hydrogenated egg phosphatidylcholine
H-EPS - Hydrogenated egg phosphatidylserine sodium
Stock solution: Trometamol (24.2 g), sodiumcalciumedetat 89% (2.8 g), hydrogen chloride 5M (to pH 7.4) and water to 100ml.

### Preparation:

A liposome concentrate is prepared by adding 300ml of a tungsten suspension (300mg/ml) to a reactor at low temperature (5-10°C). The lipids are dispersed in the cold liquid by stirring. The mixture is heated to 75-80°C and stirred for approximately half an hour, and then pumped through the homogenising cell at sufficient speed, typically 70 ml/min at a homogeniser speed of 10000-13000 rpm. The mixture is transferred to pressurized tank with heating jacket at about 80°C and pressed through an extruder with seven 1 µm polycarbonate filters at a pressure of about 2.5 bar.

The liposomes are isolated by sentrifugation. The concentrate is diluted with stock solution to a final tungsten concentration of about 160 mg tungsten/ml solution for injection.

### Example 8

### Preparation of liposomes containing tungsten particles by the thin film hydration method

A total of 100 mg of the lipids DPPC:DPPG 10:1 (10 mg/ml) are dissolved in 10 ml chloroform:methanol solvent mixture (2:1). The solution containing the dissolved lipid are introduce into a 500ml round bottomed flask and are subsequently evaporated until a dry lipid film is deposited on the wall of the flask by using a rotary evaporator. The evaporation is continued for 1 hour after the dry residue first appears, to remove traces of organic solvents.

The dried lipids are hydrated by adding 10 ml of a buffered tungsten suspension (Ex. 1-6) (300 mg/ml) to the lipid film. The flask is then rotated at about 100 r.p.m, at atmospheric pressure and for about 2 hours at a temperature of about 10°C above the transition temperature of the lipids.

The size of the liposomes is reduced by membrane extrusion by passing them through a membrane filter of 100 nm 10 times.

### Example 9

### Preparation of liposomes containing tungsten particles by the reverse-phase evaporation vesicle (REV) method

Reverse-phase evaporation vesicles (REVs) are prepared by dissolving in total 100 mg of the lipids DPPC:DPPG 10:1 in 7.5 ml chloroform:methanol solvent mixture (2:1). Five ml of tungsten suspension (Ex. 1-6)(300 mg/ml) is added to the solution during continuous stirring. The resulting solution is dried gently using a rotary evaporator at 37 °C and low vacuum (approx 600 mbar) until an intermediate gel is formed. The drying is continued until the gel reverses into a liposome suspension.

After the gel has reversed and a liposome suspension has been formed the drying is continue drying for another 10-20 minutes to remove traces of organic solvents.

Untrapped tungsten is separated from the liposomes by size exclusion chromatography.

## Claims

1. A liposome **characterized by** comprising particles comprising a core of the metallic element tungsten optionally together with other metallic elements and being coated with a coating layer, wherein the average diameter of the liposomes is between 200 and 1000 nm.

2. A liposome of claims 1 wherein the liposome comprises a lipopolysaccharides and/or lipoteichoic acid.

3. A liposome according to the preceding claims wherein the surface of the liposome comprises negative charges.

4. A liposome of the preceding claim wherein the liposome comprises negatively charged lipids.

5. A liposome of the preceding claim wherein the negatively charged lipids are negatively charged phospholipids.

6. A liposome of the preceding claims wherein the liposomes comprises neutral lipids.

7. A liposome of the preceding claims wherein the particles are of a diameter above 3 nm, preferably in the range of 3 nm to 100 nm, more preferred in the range of 3 nm to 20 nm.

8. A liposome as claimed in any of the preceding claims wherein the core of the particle has a tungsten content of 20 to 100 weight% of metallic tungsten, preferably a tungsten content of 50 to 100 weight% of metallic tungsten, more preferably 85 to 100 weight% of metallic tungsten, most preferably a tungsten content of 95 to 100 weight% of metallic tungsten.

9. A liposome as claimed in any of the preceding claims wherein the core of the particle comprises metallic tungsten and one or more of the elements rhenium, iridium, niobium, tantalum or molybdenum in their metallic form.

10. A liposome as claimed in any of the preceding claims wherein the coating of the particles comprises a polymeric coating layer.

11. A diagnostic agent comprising liposomes of claims 1 to 10 optionally together with a solvent or excipient.

12. Use of liposomes of claims 1-10 as contrast agents for the in vivo imaging of Kupffer cells.

13. A method of imaging, specifically X-ray imaging, comprising administration of liposomes of claims 1 to 10 to a human or animal body, imaging the body with an imaging device, compiling data from the examination and optionally analysing the data.

14. A process for the preparation of liposomes of claims 1 to 10 comprising the extruding method, the thin film hydration method or the reverse phase evaporation method.

## Patentansprüche

1. Liposom, **gekennzeichnet durch** das Umfassen von Teilchen, die einen Kern aus dem metallischen Element Wolfram, gegebenenfalls zusammen mit anderen metallischen Elementen, aufweisen und mit einer Überzugsschicht überzogen sind, wobei der durchschnittliche Durchmesser der Liposome zwischen 200 und 1000 nm beträgt.

2. Liposom nach Anspruch 1, wobei das Liposom Lipopolysaccharid und/oder Lipoteichonsäure umfasst.

3. Liposom nach den vorstehenden Ansprüchen, wobei die Oberfläche des Liposoms negative Ladungen aufweist.

4. Liposom nach dem vorstehenden Anspruch, wobei das Liposom negativ geladene Lipide aufweist.

5. Liposom nach dem vorstehenden Anspruch, wobei die negativ geladenen Lipide negativ geladene Phospholipide sind.

6. Liposom nach den vorstehenden Ansprüchen, wobei die Liposome neutrale Lipide umfassen.

7. Liposom nach den vorstehenden Ansprüchen, wobei die Teilchen einen Durchmesser über 3 nm, bevorzugt im Bereich von 3 nm bis 100 nm und besonders bevorzugt im Bereich von 3 nm bis 20 nm aufweisen.

8. Liposom nach einem der vorstehenden Ansprüche, wobei der Kern des Teilchens einen Wolframgehalt von 20 bis 100 Gew.-% metallischen Wolframs besitzt, bevorzugt einen Wolframgehalt von 50 bis 100 Gew.-% metallischen Wolframs, besonders bevorzugt von 85 bis 100 Gew.-% metallischen Wolframs, ganz besonders bevorzugt einen Wolframgehalt von 95 bis 100 Gew.-% metallischen Wolframs.

9. Liposom nach einem der vorstehenden Ansprüche, wobei der Kern des Teilchens metallisches Wolfram und eines oder mehrere der Elemente Rhenium, Iridium, Niob, Tantal oder Molybdän in ihrer metallischen Form umfasst.

10. Liposom nach einem der vorstehenden Ansprüche, wobei der Überzug der Teilchen eine polymere Überzugsschicht umfasst.

11. Diagnostisches Mittel mit Liposomen nach Ansprüchen 1 bis 10 gegebenenfalls zusammen mit einem Lösungsmittel oder Exzipienten.

12. Verwendung von Liposomen nach Ansprüchen 1 bis 10 als Kontrastmittel für die *in vivo* Bildgebung von Kupffer-Zellen.

13. Bildgebungsverfahren, insbesondere Röntgenbildgebung, umfassend die Verabreichung von Liposomen nach Ansprüchen 1 bis 10 an einen Menschen- oder Tierkörper, die Bildgebung vom Körper mit einer Bildgebungsvorrichtung, das Zusammenstellen von Daten aus der Untersuchung und gegebenenfalls das Analysieren der Daten.

14. Verfahren zur Herstellung von Liposomen nach Ansprüchen 1 bis 10, umfassend das Extrusionsverfahren, das Dünnfilmhydrationsverfahren oder das Umkehrphasenverdampfungsverfahren.

## Revendications

1. Liposome **caractérisé par le fait qu'**il comprend des particules comprenant un noyau de l'élément métallique tungstène éventuellement ensemble avec d'autres éléments métalliques et qui est revêtu d'une couche de revêtement, dans lequel le diamètre moyen des liposomes est compris entre 200 et 1000 nm.

2. Liposome selon la revendication 1, dans lequel le liposome comprend des lipopolysaccharides et/ou de l'acide lipoteichoïque.

3. Liposome selon les revendications précédentes, dans lequel la surface du liposome comprend des charges négatives.

4. Liposome selon les revendications précédentes, dans lequel le liposome comprend des lipides chargés négativement.

5. Liposome selon les revendications précédentes, dans lequel les lipides chargés négativement sont des phospholipides chargés négativement.

6. Liposome selon les revendications précédentes, dans lequel les liposomes comprennent des lipides neutres.

7. Liposome selon les revendications précédentes, dans lequel les particules sont d'un diamètre supérieur à 3 nm, de préférence, dans la plage de 3 nm à 100 nm, plus préférablement dans la plage de 3 nm à 20 nm.

8. Liposome selon l'une quelconque des revendications précédentes, dans lequel le noyau de la particule présente une teneur en tungstène de 20 à 100 % en poids de tungstène métallique, de préférence, une teneur en tungstène de 50 à 100 % en poids de tungstène métallique, plus préférablement de 85 à 100 % en poids de tungstène métallique, encore plus préférablement, une teneur en tungstène de 95 à 100 % en poids de tungstène métallique.

9. Liposome selon l'une quelconque des revendications précédentes, dans lequel le noyau de la particule comprend du tungstène métallique et un ou plusieurs des éléments rhénium, iridium, niobium, tantale ou molybdène sous leur forme métallique.

10. Liposome selon l'une quelconque des revendications précédentes, dans lequel le revêtement des particules comprend une couche de revêtement polymère.

11. Agent de diagnostic comprenant des liposomes selon les revendications 1 à 10, éventuellement ensemble avec un solvant ou un excipient.

12. Utilisation de liposomes selon les revendications 1 à 10 en tant qu'agent de contraste pour l'imagerie in vivo de cellules de Kupffer.

13. Procédé d'imagerie, en particulier, de radiographie, comprenant l'administration de liposomes selon les revendications 1 à 10 dans un corps humain ou animal, l'imagerie du corps avec un dispositif d'imagerie, la compilation des données à partir de l'examen et, en variante, l'analyse des données.

14. Procédé de préparation de liposomes selon les revendications 1 à 10, comprenant le procédé d'extrusion, le procédé par hydratation de film mince ou le procédé par évaporation en phase inversée.
